# EUROPEAN PATENT SPECIFICATION

(11) **EP 1 281 087 B1**
(45) Date of publication and mention of the grant of the patent: **09.01.2008**
(21) Application number: 01949312.1
(22) Date of filing: 04.05.2001
(51) Int. Cl.: G01N 33/574, C12Q 1/68

(54) **EARLY DIAGNOSIS OF BLADDER TUMOR IN URINE SAMPLES**
FRÜHEN DIAGNOSE VON BLASENTUMOR IN URINPROBEN
DIAGNOSTIC PRECOCE D'UNE TUMEUR DE LA VESSIE DANS DES PRELEVEMENTS D'URINE

(30) Priority: 08.05.2000 IT MI001002
(43) Date of publication of application: 05.02.2003
(73) Proprietor: Macrochip S.R.L., 34100 Trieste (IT)
(72) Inventor: STANTA, Giorgio, I-34011 Duino Aurisiana (IT)
(74) Representative: Modiano, Micaela Nadia
(86) International application number: PCT/EP2001/005054
(87) International publication number: WO 2001/086288

(56) References cited:
- EP-A- 0 841 396
- EP-A- 0 926 245
- EP-A- 0 930 369
- WO-A-97/35871
- US-A- 5 863 726
- DE KOK, JACQUES B. (1) ET AL: "Real-time quantification of human telomerase reverse transcriptase mRNA in tumors and healthy tissues." CLINICAL CHEMISTRY, (MARCH, 2000) VOL. 46, NO. 3, PP. 313-318. , XP002176598
- BIALKOWSKA-HOBRZANSKA, HANNA ET AL: "Comparison of human telomerase reverse transcriptase messenger RNA and telomerase activity as urine markers for diagnosis of bladder carcinoma" MOL. DIAGN. (2000), 5(4), 267-277 , XP008000978
- MUELLER, MARKUS (1) ET AL: "Diagnosis of bladder cancer by detecting: Different telomerase subunits in urine." JOURNAL OF CLINICAL LIGAND ASSAY, (WINTER, 1999) VOL. 22, NO. 4, PP. 354-357. , XP001029128

## Description

### Technical Field

The present invention relates to a method and an apparatus for early diagnosis of bladder tumor in urine samples.

In particular, the present invention relates to a method and to the corresponding diagnostic apparatus for detecting, from the onset, even initial tumor forms affecting the mucous membrane of the bladder without resorting to the use of more or less invasive methods.

The method according to the invention is particularly suitable for detecting transitional carcinomas of the bladder, which make up almost all of the tumors affecting this region.

### Background art

It is known that transitional bladder cell carcinoma (TCC) is one of the most widespread tumor in Western societies. At the time of diagnosis, 20% of TCCs are found to be of the invasive type, while 80% are superficial. 20% of the latter can evolve into an invasive form with infiltration of the muscular tunic of the bladder wall.

Due to the frequent relapses of these forms of tumor, periodic monitoring of patients, including asymptomatic ones, is indispensable.

However, it has been found that cystoscopy, which is currently the most widely used monitoring method because it provides the most reliable results, cannot be considered a method suitable for the screening of a large number of patients due to invasive nature of the procedure, which is particularly unpleasant in male patients.

An alternative non-invasive diagnostic method is cytological examination of urine. This method has the disadvantage of low sensitivity, which allows to recognize the neoplastic lesion only in 40-50% of investigated cases.

Transitional bladder tumor usually affects the elderly population; which is increasing considerably in developed societies. This leads to the expectation of an increase in the number of requests for diagnosis of these tumors in the future. Since the analyses to which patients are subjected for accurate diagnosis are mostly invasive, an alternative, noninvasive, high-sensitivity and high-specificity method would be very important. Cytology, which is a noninvasive method, has low sensitivity and is also affected by the variability of the interpretation of the people who perform the test. Commercially available alternative methods are based on determining the various tumor-specific antigens (BTA, bladder tumor associated analytes (Bard Diagnostics, Redmond, Washington), NMP 22 nuclear matrix protein [Matritech, Newton, Massachusetts]). Although these methods are more sensitive than cytological examination, their stated clinical usefulness is compromised by the extreme variability due to the inflammatory processes, which can interfere with their sensitivity and specificity.

It is known that molecular techniques, based on DNA and RNA analysis, are extremely sensitive and specific and are therefore suggested increasingly also in clinical diagnostics.

It is well-known that in bladder tumor the pattern of genetic alterations, which cause the development of neoplastic cells, is highly complex, making analysis particularly troublesome. One of the first mutations studied at the level of tumor cells (ras oncogene mutation) has been found in bladder tumor cell lines and rarely in primitive bladder tumor.

Sidransky et al. (Science (1991) 252, 252-253) found point mutations of the oncosuppressor gene p53 in 61% of bladder tumor cases. Due to this relatively low frequency of mutations and to the large number of variations in said mutations, this investigation method cannot be used in clinical diagnostics.

Cytogenetic methods have revealed some chromosomal deletions at 9q, 11p and 17p, where oncosuppressor genes may be located. In order to identify loss of heterozygosity and gene instability, microsatellite markers have an important application. These genetic alterations at the microsatellite level, however, are occasional, and in a significant number of tumors might not be revealed despite using a large number of microsatellite markers. Mao et al. (Science (1996) 271, 659-662) used a group of 13 tri- and tetranucleotide markers (it has been found that the longest repeats are those subjected to deletions or expansion). 95% of tumor cases (19 out of 20 cases) had an identical clonal alteration in the urine sample and in tumor biopsies.

Although the method has been found to be sensitive, it is not suitable for screening because it is extremely labor-intensive (large number of individuals to be analyzed).

The following diagnostic kits are currently commercially available:
-- BTA (bladder tumor associated analytes), which has 74% sensitivity and 73% specificity. False positives have been found in patients without a bladder tumor but with considerable hematuria (6 out of 7 cases).
-- NMP22 (nuclear matrix protein), which has 53% sensitivity and 60% specificity.
-- FDP (fibrin and fibrinogen degradation products), which has 52% sensitivity and 91% specificity (The Journal of Urology (1999) 161, 388-394).

Currently, the need is felt to have a new method, with better characteristics, for the diagnosis and for following the evolution of bladder tumor, which is highly sensitive and specific, does not require harmful reagents and can be used directly on the urine of patients.

Determination of telomerase activity in cells obtained by bladder washing has recently been proposed (Clin. Canc. Research. (1998) 4, 535-538). Telomeres are the terminal portions of eukaryotic chromosomes and are meant to protect and stabilize chromosomes. Since DNA polymerase is unable to synthesize them during replication of somatic cells, telomeres gradually shorten. When the telomeres reach a limit length, chromosomal instability leads to cell death (apoptosis). In immortalization of somatic cells and in malignant transformation, the telomeres stabilize and telomerase enzyme activity appears.

Telomerase is the enzyme responsible for telomere synthesis. It is constituted by three components (Current opinion in Genetics and Development (1999) 9, 97-103):
1) the RNA (hTR) component, which acts as a template for telomere synthesis (short repetitive sequences);
2) the catalytic component - hTRT;
3) the telomerase-associated protein (TEP1).

The presence of the two components hTR and hTRT is sufficient to reconstruct telomerase activity in vitro.

Telomerase activity (addition of telomere repeats at the end of chromosomes, which occurs by using as template its RNA component [hTR]) is measured with the TRAP assay (telomeric repeat amplification protocol). Boehringer Mannheim (Roche Biochemicals) has developed a nonradioactive TRAP analysis system based on a method which uses an ELISA-type microplate. Since in excessively concentrated protein lysates the presence of Taq polymerase inhibitors may mask telomerase activity (false negatives), the TRAP assay must be performed by using various dilutions of the same sample. Furthermore, the presence of protease, RNase and the pH variations to which cells are exposed in urine can compromise the efficiency of the assay (false negatives).

Lee et al. (Clin. Can. Res. (1998) 4, 535-538) have demonstrated telomerase activity (TRAP assay) in 96% of tumor cases by performing analysis on tumor tissue and in bladder washes. Specificity is 96%. Heine (Journal of Pathology (1998) 184, 71-76) et al. have shown, with TRAP analysis, telomerase activity in 95% (19 out of 20) of bladder tumor tissues and in 70% (14 out of 20) of bladder washes, while activity was absent in urine sediment of bladder tumor patients. Rahat et al. (Cancer (1999) 85, 919-924) have demonstrated telomerase activity with TRAP analysis in 81% (17 out of 20) of urine samples of bladder tumor patients, with a specificity of 76%.

It has been demonstrated that telomerase activity is proportional to the quantity of messenger RNA of the protein component of telomerase (hTRT) (FEBS Letter (1999) 460, 285-288). Ito et al. (Clin. Can. Res. (1998) 4, 2870, 2810) have demonstrated that hTRT mRNA, the catalytic part of telomerase, is expressed in urine sediment in 76% (23 out of 33) of bladder tumor cases. The specificity of the method is 96%. Ito et al. (Clin. Can. Res. (1998) 4, 1603-1608) have provided studies on the distribution of the expression of all three components of telomerase, i.e., hTRT, hTR and TEP1, in bladder tumor tissues. hTRT is expressed in 90% of tumor tissues (30 out of 33) and in 20% of neighboring tissues. On the contrary, hTR and TEP1 are expressed both in tumor tissues and in normal tissues. Scientific literature has found no significant correlation between expression of hTRT and clinical-pathological characteristics of the tumors. Suzuki et al. (The Journal of Urology (1999) 162, 2217-2220) have demonstrated that hTRT is expressed in 100% of bladder tumor tissues (27 cases) and in none of the normal tissues that surround the tumor, while the TEP1 protein is expressed equally in tumor tissues and in normal tissues.

Medline abstract NLM9817302; BUCHUMESKY, V ET AL:"Cytokeratin 20: a new marker for early detection of bladder cell carcinoma?" The Journal of Urology (1998), 160(6) Pt 1, 1971-1974, teaches that CK-20 is a bladder tumour marker in urine.

### Disclosure of the Invention

A noninvasive method for early diagnosis of bladder tumor has now been found which is based on the simultaneous determination of the expression of the catalytic part of telomerase (hTRT) at the level of messenger RNA and of at least one additional selected molecular marker.

The diagnostic method is based on the simultaneous determination of the messenger RNA of the protein component of telomerase, which is a marker for cell immortalization and therefore for the presence of cells in vivo which have undergone a malignant transformation, and of another selected marker.

In particular, the diagnostic method is based on highlighting both the expression of the telomerase enzyme by quantization of the messenger RNA of the catalytic part (hTRT marker) in urine and the expression of at least one additional molecular marker, and on subsequent quantization thereof and analysis of the results obtained.

The method based on the interpretation of the response of two or more molecular markers increases sensitivity and specificity up to 100% of both. In this manner, the method according to the invention is suitable for screening and monitoring of patients undergoing treatment.

In accordance with a first aspect of the present invention, a diagnostic method for bladder tumor is provided which comprises a step of amplification of the RNA extracted from the cells present in a urine sample by using:
-- an hTRT marker;
-- a marker, preferably β-actin, as demonstration of RNA accessibility and as standard for quantitative estimation,
in association with an other marker that is a marker for lymphocytes which is suitable to detect inflammatory cells associated with neoplastic infiltration, and optionally a marker for one of the proteins of the cytokeratin family;
said protein marker of the cytokeratin family being cytokeratin 20 or CK20 and said lymphocyte marker suitable to detect inflammatory cells associated with the neoplastic infiltration being CD4.

In particular, the present invention provides a method for early diagnosis of bladder tumor in a urine sample which comprises:
a) optionally, a preliminary step of extracting the total RNA from the cells that are present in the urine;
b) a step of amplification of the extracted RNA by using:
   -- a marker for the messenger RNA of the catalytic component of telomerase (hTRT);
   -- a marker for β-actin
   in combination with an additional marker that is a lymphocyte marker which is suitable to detect inflammatory cells associated with neoplastic infiltration; and optionally a marker for a protein of the cytokeratin family;
   and, advantageously, a thermocycler or real-time PCR,
   said protein marker of the cytokeratin family being cytokeratin 20 or CK20 and said lymphocyte marker suitable to detect inflammatory cells associated with the neoplastic infiltration being CD4,
c) a step of detecting the amplified material.

One of the markers used in the invention is cytokeratin 20 (CK20) which belongs to the cytokeratin family, which is composed of 20 polypeptides which are part of the intermediate filaments of epithelial cells. Cytokeratins are expressed in various combinations according to the type of epithelial cell and to the degree of differentiation.

Furthermore, it has been found that among markers for lymphocytes, CD4 which is used in the present invention is particularly suitable to detect the presence of helper lymphocytes associated with neoplastic infiltration.

In particular, determination of the RNA of hTRT in combination with three other markers, such as cytokeratin 20, CD4 and β-actin, and subsequent coordinated interpretation of the results provide a means for a sensitive and specific diagnosis of bladder tumor directly in urine.

The step for amplification of extracted RNA according to the method of the invention advantageously comprises two steps:
i) a reverse transcription (RT) step, performed by providing a specific antisense primer or primers and
ii) an amplification step (PCR), using the respective specific sense primers, which are advantageously biotinylated in order to facilitate the subsequent detection step.

In accordance with one embodiment, the amplification step is performed with a thermocycler or real-time PCR.

According to another embodiment of the invention, the amplification step is performed in the presence of ddUTP-biotinylate.

The subsequent revelation step comprises:
-- hybridization of the products amplified in step b) in a first well which contains a probe for the amplified hTRT marker and at least one well which contains a probe for a marker for a protein of the cytokeratin family, a marker for a lymphocyte suitable to detect inflammatory cells associated with neoplastic infiltration, and β-actin. Alternatively, the biotinylated amplified material is bound to the plate with streptavidin and the amplification products are hybridized with the probe, preferably marked with DIG.
-- detection of the bound fragment (recognition that hybridization has occurred) with the probe used, which is preferably performed by using streptavidin, which recognizes biotin and with which alkaline phosphatase or peroxidase is advantageously bound.

In accordance with another embodiment, the amplified material, hybridized with the probe marked with DIG, is detected with anti-DIG with which alkaline phophatase or peroxidase is advantageously bound. Instrumental detection, in both cases (for example with an ELISA reader), is preferably performed with the addition of a colorimetric substrate or by chemiluminescence.

As an alternative to alkaline phosphatase or peroxidase, streptavidin is marked with molecules for fluorescence emission.

In the case of use of real-time PCR, which is capable of performing amplification in real time, the detection system used can be an intercalating staining solution, such as Syber green or specific probes marked with molecules for fluorescence emission.

The diagnostic method according to the invention allows to identify neoplastic cells of epithelial origin and CD4+ lymphocytes. In particular, the combination of markers according to the invention allows to obtain detailed information regarding the desquamation of neoplastic uroepithelial cells and the infiltration of the tumor on the part of CD4+ lymphocytes. The method according to the invention allows to analyze the cells that are present in the urine even if they are not perfectly preserved, differently from the TRAP method, which leads to a false negative if the proteins are degraded.

The method according to the invention also avoids the need to perform bladder washing, since the urine of the patient can be used directly.

This last advantage is very important, since bladder washing is in any case a diagnostic intervention performed on the patient, with additional costs and discomfort for said patient.

The method according to the invention provides a particularly accurate indication of the existence of a malignant tumor form in progress, located at the bladder, and also provides considerable advantages with respect to the other methods used so far.

According to another aspect of the invention, an apparatus for the diagnosis or for monitoring bladder tumor is provided which comprises:
-- a marker for the messenger RNA of the catalytic component of telomerase (hTRT), a marker for β-actin to demonstrate RNA accessibility and as standard for quantitative estimation, and an additional marker that is a marker for a lymphocyte suitable to detect inflammatory cells associated with neoplastic infiltration, and optionally a marker for a protein of the cytokeratin family;
   said marker for a lymphocyte suitable to detect inflammatory cells associated with neoplastic infiltration being CD4, and said marker for a protein of the cytokeratin family being cytokeratin 20 or CK20.

Advantageously, the apparatus according to the invention furthermore comprises a plurality of ELISA plates and conveniently one or more of the above described reagents, markers, and probes.

The following examples are provided merely as a non-limitative illustration of the present invention.

### Example 1

### A) Urine collection and storage

A human urine sample was processed according to the following procedure directly after collection:
-- collection of urine sediment by passing the urine to be tested through an appropriate filter, with application of vacuum (Talent srl);
-- the filter containing the cells was immersed in a lysis solution ("Total Quick RNA" kit by Talent), centrifuged for 1 minutes, and left immersed for 10 minutes.

The solution, after removing the filter, was stored at -80 °C.

### B) Extraction of total RNA.

Extraction was performed (by means of the "Total Quick RNA" method by Talent, Trieste, Italy) by using the specific column. The RNA bound selectively with the resin, which was subsequently washed to eliminate traces of protein and DNA. The RNA was then eluted with warm water.

C) Diagnostic investigation according to the invention.

The method was conducted on an ELISA-type microplate.
I) Step of amplification of extracted RNA. This step was performed by using four specific markers and a thermocycler.
   Amplification occurred in two steps:
   1) reverse transcription by using a specific antisense primer
   2) amplification by using the respective biotinylated specific sense primers (for the subsequent revelation procedure).
II) a detection step.
   The amplification products were hybridized in four different wells. Each well contained one of the four specific probes: telomerase, CK20, CD4 and β-actin. For each marker, positive and negative control amplification was performed, with subsequent hybridization on a microplate. The probe was complementary to the fragment amplified with the biotinylated primer. Detection of the bound fragment occurred by means of streptavidin (which recognizes biotin), to which alkaline phosphatase was bound. After adding the colorimetric substrate (4-nitrophenyl phosphate) or chemiluminescence substrate, reading occurred with the appropriate instrument (for example with an ELISA reader).

The streptavidin was alternatively bound with a fluorochrome which was detected by virtue of an appropriate system (for example with an ELISA reader).

The cutoff levels of the CD4 and CK20 markers were chosen from the first series of experiments, using the combination of three markers (hTRT, CK20 and CD4) in a small number of cases with neoplasms and phlogosis. The cases that expressed even a minimal quantity of hTRT were considered positive. All the markers were compared with the values of β-actin.

### Interpretation of results:

The cases in which two or three markers were positive (in the case of CK20 and CD4, above the preset threshold) were considered positive (+) for tumor. Using this approach, the tumors (27 out of 31) that had already been diagnosed at the clinical level and the negative cases that also reflected the clinical diagnosis of cystitis were defined.

The cases in which only one marker of the three proposed ones (hTRT, CK20 and CD4) was positive were defined as inconclusive (10 cases). Of these, 40% (4 cases) were found to be tumors (comparing the results with clinical results) and 60% were clinically negative.

Since the tumor cases defined as inconclusive are proposed for subsequent retesting, the method according to the invention has 100% sensitivity (27 tumor cases diagnosed with our method + 4 cases defined as inconclusive). The specificity of the method was 45% (5 cases of cystitis out of 11), but if the inconclusive cases are considered, specificity is 100%. In other words, a tumor was never defined as negative and a cystitis was never considered positive.

Interpretation of the results allowed to define 3 groups:
1) Positive for at least two markers -- tumor (all cases were found to be real tumors)
2) Negative for all three markers -- absence of tumor (none of the cases defined as negative had neoplasms)
3) Inconclusive cases because only one of the markers is positive: investigation to be repeated or confirmed with other methods (of the 10 cases defined as inconclusive, 4 were then found to be tumor).

Considering the sensitivity and specificity values expressed by the individual markers, the following values were found:
1) For hTRT: 94% sensitivity, 82% specificity
2) For CK20: 74% sensitivity, 64% specificity
3) For CD4: 52% sensitivity and 100% specificity

The following Table 1 is an interpretation of the quantitative results on 42 patients (31 with tumor) expressed as sensitivity and specificity. The specificity and sensitivity of our method are compared with other noninvasive methods.

**TABLE 1**

| Analysis | Sensitivity | Specificity |
|---|---|---|
| hTRT marker | 94% | 82% |
| CK20 marker | 74% | 64% |
| CD4 marker | 52% | 100% |
| Markers: hTRT + CK20 + CD4 + inconclusive cases (after second analysis performed on inconclusive cases by taking a new sample of the same patient) | 100% | 100% |
| TRAP | 70% | 99% |
| BTA | 74% | 73% |
| NMP22 | 53% | 62% |
| Cytology | 44% | 95% |
| FDP | 52% | 95% |

The following Table 2 lists the results found in 42 cases with neoplasms and phlogosis. The results show that the cases found positive simultaneously for the hTRT marker, the cytokeratin marker and the lymphocyte marker according to the present invention led to a diagnosis of bladder tumor which was confirmed clinically, indicating a high reliability of the diagnostic results found with the method according to the present invention.

**TABLE 2**

| Cases | hTRT | CD4 | CK20 | Diagnosis with our method | Clinical diagnosis |
|---|---|---|---|---|---|
| 1 | + | - | + | Tumor | Tumor |
| 2 | - | - | - | Negative | Cystitis |
| 3 | + | + | + | Tumor | Tumor |
| 4 | - | - | + | Inconclusive | Cystitis |
| 5 | - | - | + | Inconclusive | Cystitis |
| 6 | - | - | - | Negative | Cystitis |
| 7 | + | - | - | Inconclusive | Tumor |
| 8 | + | + | - | Tumor | Tumor |
| 9 | + | - | + | Tumor | Tumor |
| 10 | - | - | - | Negative | Cystitis |
| 11 | + | - | + | Tumor | Tumor |
| 12 | + | - | + | Tumor | Tumor |
| 13 | + | - | + | Tumor | Tumor |
| 14 | + | + | - | Tumor | Tumor |
| 15 | - | - | - | Negative | Cystitis |
| 16 | - | - | + | Inconclusive | Cystitis |
| 17 | - | - | + | Inconclusive | Cystitis |
| 18 | + | - | + | Tumor | Tumor |
| 19 | + | - | - | Inconclusive | Tumor |
| 20 | - | - | + | Inconclusive | Tumor |
| 21 | + | - | + | Tumor | Tumor |
| 22 | + | - | + | Tumor | Tumor |
| 23 | + | - | - | Inconclusive | Cystitis |
| 24 | + | - | + | Tumor | Tumor |
| 25 | + | + | + | Tumor | Tumor |
| 26 | + | + | + | Tumor | Tumor |
| 27 | + | - | + | Tumor | Tumor |
| 28 | + | + | - | Tumor | Tumor |
| 29 | + | + | - | Tumor | Tumor |
| 30 | + | + | - | Tumor | Tumor |
| 31 | + | + | + | Tumor | Tumor |
| 32 | + | + | + | Tumor | Tumor |
| 33 | + | + | + | Tumor | Tumor |
| 34 | + | + | + | Tumor | Tumor |
| 35 | + | - | - | Inconclusive | Cystitis |
| 36 | + | + | - | Tumor | Tumor |
| 37 | - | + | + | Tumor | Tumor |
| 38 | - | + | + | Tumor | Tumor |
| 39 | + | - | + | Tumor | Tumor |
| 40 | + | - | + | Tumor | Tumor |
| 41 | - | - | - | Cystitis | Cystitis |
| 42 | - | + | - | Inconclusive | Tumor |

### Example 2.

Reagents and instruments used to provide an embodiment of the method according to the invention:

### For RNA extraction

--absolute ethanol
-- 75% ethanol
-- microcentrifuge
-- sterile tips

### For RT-PCR

-- thermocycler

### For detection

-- ELISA reader

Content of an embodiment of a diagnostic kit:
1) Accessories for collecting urine sediment
2) Kit for RNA extraction
3) Kit for RT-PCR
4) Detection kit

### Kit for RNA extraction

TQ-RNA Lysing solution
TQ-RNA Binding resin
TQ-RNA Washing solution A
TQ-RNA Washing solution B
TQ-RNA Conditioning solution
TQ-RNA Spin columns
2 ml collection tubes
1.5 collection tubes

### Accessories for collecting urine sediment

Urine collection filters
vacuum manifold

### Kit for RT-PCR

Primer up (biotinylated)
Primer down
Reverse transcriptase
5x buffer for reverse transcriptase
10x buffer for PCR
Taq polymerase

### Kit for detection

-- ELISA plates (4 plates, each containing one of the four specific probes: telomerase, CK20, CD4 and β-actin)
-- Reagent 1 (blocking reagent)
-- Reagent 2 (hybridization solution)
-- Reagent 3A (washing solution)
-- Reagent 3B (washing solution)
-- Streptavidin, to which alkaline phosphatase is bound
-- Reagent 4
-- Reagent 5 (washing solution)
-- Reagent 6 (substrate solution)
-- Colorimetric substrate (4-nitrophenyl phosphate)
-- Reagent 7

### Urine collection and storage

The urine samples must be processed as quickly as possible:
4) urine sediment was collected by vacuum and appropriate filters (supplied by Talent).
5) the filter containing the cells was immersed in the lysing solution ("Total Quick RNA" kit by Talent), centrifuged for 1 minute, and left immersed for 10 minutes.
6) the solution, after removing the filter, was stored at -80°C.

### Extraction of total RNA with the "Total Quick RNA" method by Talent:

Extraction was performed with the "Total Quick RNA" method by Talent, which is a quick method (20 minutes) based on column centrifugation. The RNA was specifically bound to the resin, which was then washed to eliminate traces of protein and DNA, and then eluted with warm water.

### RT-PCR

RNA concentration was measured at 260 nm absorbance. Absorbance equal to 1 unit corresponds to 40 µg of RNA per ml.

The step for amplification of the extracted RNA by using four specific markers and a thermocycler occurred in two steps:
1) reverse transcription by using a specific antisense primer (RT)
2) amplification (PCR) by using the respective specific sense primers which are biotinylated (for subsequent revelation of bound fragments).

### RT

### Preparation of mix:

| | |
|---|---|
| 5x buffer | 2µl |
| Primer down (30 pmol/µl) | 0.5 µl |
| Mixture of NTPs | 1 µl |
| AMV | 0.1 µl |
| RNase | 0.1 |
| H₂O | 5.3 µl |
| RNA (500 ng/µl) | 1 µl |
| Total volume | 10 µl |

### Incubation at 42°C was performed for 1 hour.

### PCR

| Preparing the mix: | |
|---|---|
| 10x buffer | 5 µl |
| Primer up (biotinylated) | 0.5 µl |
| Taq polymerase | 0.25 µl |
| H₂O | 34.25 µl |
| Total volume | 40 µl |

The mix was added in the respective tubes of the RT and amplified by performing the following cycles:
95 °C for 3 minutes
5 cycles of:
95 °C for 1 minute
55°C for 1 minute
72 °C for 1 minute
40 cycles of:
95 °C for 30 seconds
55 °C for 30 seconds
72 °C for 30 seconds

### Detection

The amplification products were adsorbed on four different wells (for example, from a total of 50 µl, 10 µl per well were placed (each well contained one of the four specific probes: telomerase, CK20, CD4 and β-actin). The probe was complementary to the fragment amplified with the biotinylated primer.

Detection of the bound fragment occurred by means of streptavidin (which recognizes biotin), to which the alkaline phosphatase was bound. After adding the colorimetric substrate (4-nitrophenyl phosphate), reading was performed with an ELISA reader.

The analysis procedure entailed performing the following steps:
1) 200 µl of Reagent 1 were added in the wells of the ELISA and incubation was performed at ambient temperature for one hour under agitation.
2) the amplified material was denatured at 95 °C for 10 minutes, placing it immediately in ice.
3) the wells were emptied. 90 µl of Reagent 2 and 10 µl of each amplified product were added in the respective wells, and incubation was performed at 50 °C for two hours.
4) The wells were emptied and washed 3 times with 200 µl of Reagent 3A heated at 50 °C.
5) The wells were incubated with Reagent 3A at 50 °C for 15 minutes.
6) Emptying and washing were performed 3 times with 200 µl of Reagent 3B heated to 50 °C.
7) For each well, 100 µl of streptavidin, conjugated with alkaline phosphatase, diluted at 1:5000 in Reagent 4, were added.
8) Incubation was performed at 50 °C for 1 hour.
9) The wells were emptied and washed 3 times with 200 µl of Reagent 5 at ambient temperature.
10) The wells were incubated with Reagent 5 for 5 minutes at ambient temperature.
11) Washing was performed 3 times with 200 µl of Reagent 5 at ambient temperature.
12) 100 µl of pNPP, 10 mg/ml, dissolved in solution 6, were added.
13) Incubation was performed for 30 minutes at ambient temperature in darkness.
14) The reaction was blocked with 100 µl of Reagent 7.
15) The results were read at 405 nm.

The disclosures in Italian Patent Application No. MI2000A001002 from which this application claims priority are refered to.

## Claims

1. A method for early diagnosis of bladder tumor in a urine sample, **characterized in that** it comprises the determination, on the RNA extracted from the cells present in the urine, of:
-- a marker for the messenger RNA of the catalytic component of telomerase (hTRT) and a marker, preferably β-actin, to demonstrate RNA accessibility and as standard for quantitative estimation, in association with an additional marker that is a lymphocyte marker which is suitable to detect inflammatory cells associated with neoplastic infiltration, said lymphocyte marker suitable to detect inflammatory cells associated with the neoplastic infiltration being CD4, and optionally a marker for a protein of the cytokeratin family, said protein marker of the cytokeratin family being cytokeratin 20 or CK20.

2. A method for early diagnosis of bladder tumor in a urine sample, comprising:
a) a step of amplification of the RNA extracted from cells present in the urine by using:
-- a marker for the messenger RNA of the catalytic component of telomerase (hTRT), a marker for β-actin to demonstrate RNA accessibility and as standard for quantitative estimation,
in combination with an additional marker that is a lymphocyte marker which is suitable to detect inflammatory cells associated with neoplastic infiltration, said lymphocyte marker suitable to detect inflammatory cells associated with the neoplastic infiltration being CD4; and optionally a marker for a protein of the cytokeratin family, said protein marker of the cytokeratin family being cytokeratin 20 or CK20;
b) a step of detecting the amplified material.

3. The method according to claim 2, **characterized in that** said amplification comprises:
i) reverse transcription by using a specific antisense primer;
ii) amplification by using specific sense primers.

4. The method according to claim 2, **characterized in that** said specific sense primers of the amplification step ii) are preferably biotinylated in order to facilitate the subsequent detection step.

5. The method according to claim 2, **characterized in that** said detection step comprises hybridization of the amplified products with probes which are specific for the markers used in said step b) and the subsequent recognition that hybridization has occurred with said probe preferably by means of streptavidin.

6. The method according to claim 5, **characterized in that** said streptavidin is conjugated with alkaline phosphatase or with peroxidase for colorimetric or chemiluminescence detection.

7. The method according to claim 5, **characterized in that** said streptavidin is conjugated with fluorescein for fluorescence detection.

8. The method according to claim 2, **characterized in that** said amplification products of step b) are adsorbed on a first well which contains a probe which is immobilized for hTRT and on at least one additional well which contains a probe for the marker used in said step b).

9. The method according to any one of the preceding claims 1 to 8, **characterized in that** it comprises a preliminary step of extracting the total RNA from the cells that are present in the urine.

10. An apparatus for diagnosis or for monitoring bladder tumor, **characterized in that** it comprises:
-- a messenger RNA marker for the catalytic component of telomerase (hTRT), a β-actin marker to demonstrate RNA accessibility and as a standard for quantitative estimation, and an additional marker that is a lymphocyte marker suitable to detect inflammatory cells associated with neoplastic infiltration, and optionally a protein marker of the cytokeratin family; said lymphocyte marker suitable to detect inflammatory cells associated with neoplastic infiltration being CD4.

11. The apparatus according to claim 10, **characterized in that** it furthermore comprises a plurality of ELISA plates.

12. The apparatus according to claim 11, **characterized in that** it comprises 4 ELISA plates, each of which comprises one of the four specific probes: telomerase, CK20, CD4, and β-actin.

13. The apparatus according to any one of claims 10 to 12, **characterized in that** it furthermore comprises streptavidin to which alkaline phosphatase or peroxidase is linked.

14. The apparatus according to any one of claims 10 to 13, **characterized in that** it furthermore comprises one or more washing solutions.

15. The apparatus according to any one of claims 10 to 14, **characterized in that** it furthermore comprises a solution for hybridization and a colorimetric substrate.

16. The apparatus according to any one of claims 10 to 12 and 14-15, **characterized in that** it furthermore comprises streptavidin conjugated with fluorescein for fluorescence detection.

## Patentansprüche

1. Ein Verfahren zur Frühdiagnose von Blasentumor in einer Harnprobe, **dadurch gekennzeichnet, dass** es von der RNA, die von im Harn vorhandenen Zellen extrahiert ist, die Feststellung von folgendem umfasst:
-- einen Marker für den Botenstoff RNA der katalytischen Komponente von Telomerase (hTRT) und einen Marker, bevorzugt β-Actin, zum Nachweisen von RNA-Zugänglichkeit und als Maßstab für quantitative Schätzung,
in Verbindung mit einem zusätzlichen Marker, der ein Lymphozytenmarker ist, der geeignet ist, Entzündungszellen festzustellen, die mit neoplastischer Infiltration assoziiert sind, wobei der Lymphozytenmarker, der geeignet ist, Entzündungszellen festzustellen, die mit neoplastischer Infiltration in Verbindung stehen, CD4 ist, und optional ein Marker für ein Protein der Cytokeratin-Familie, wobei der Proteinmarker Cytokeratin 20 oder CK20 aus der Cytokeratinfamilie ist.

2. Ein Verfahren zur Frühdiagnose von Blasentumor in einer Harnprobe, umfassend:
a) ein Schritt zur Verstärkung der RNA, die von im Harn vorhandenen Zellen extrahiert ist, unter Verwendung von:
einem Marker für den Botenstoff RNA der katalytischen Komponente von Telomerase (hTRT), einem Marker für β-Actin, um RNA-Zugänglichkeit nachzuweisen und als Maßstab für quantitative Schätzung,
in Kombination mit einem zusätzlichen Marker, der ein Lymphozytenmarker ist, der geeignet ist, Entzündungszellen, die mit neoplastischer Infiltration assoziiert sind, festzustellen, wobei der Lymphozytenmarker, der geeignet ist, die Entzündungszellen festzustellen, die mit neoplastischer Infiltration assoziiert sind, CD4 ist; und optional, ein Marker für ein Protein der Cytokeratin-Familie, wobei der Proteinmarker Cytokeratin 20 oder CK20 aus der Cytokeratinfamilie ist;
b) einem Schritt zum Feststellen des verstärkten Materials.

3. Das Verfahren gemäß Anspruch 2, **dadurch gekennzeichnet, dass** die Verstärkung umfasst:
i) Reverse Transkription durch die Verwendung eines spezifischen Antisense-Primer;
ii) Verstärkung durch die Verwendung spezifischer Sense-Primer.

4. Das Verfahren gemäß Anspruch 2, **dadurch gekennzeichnet, dass** die spezifischen Sense-Primer des Verstärkungsschrittes ii) bevorzugt biotinyliert sind, um den nachfolgenden Feststellungsschritt zu ermöglichen.

5. Das Verfahren gemäß Anspruch 2, **dadurch gekennzeichnet, dass** der Feststellungsschritt Hybridisierung der verstärkten Produkte mit Proben, die für die in dem Schritt b) benutzten Marker spezifisch sind und die nachfolgende Erkennung, dass Hybridisierung mit der Probe aufgetreten ist, vorzugsweise mittels Streptavidin, umfasst.

6. Das Verfahren gemäß Anspruch 5, **dadurch gekennzeichnet, dass** das Streptavidin mit alkalischer Phosphatase oder mit Peroxydase zur kolorimetrischen oder chemilumineszenten Feststellung konjugiert ist.

7. Das Verfahren gemäß Anspruch 5, **dadurch gekennzeichnet, dass** das Streptavidin mit Fluoreszin zur Fluoreszenzfeststellung konjugiert ist.

8. Das Verfahren gemäß Anspruch 2, **dadurch gekennzeichnet, dass** die Verstärkungsprodukte von Schritt b) an einer ersten Mulde, die eine Probe enthält, die für hTRT immobilisiert ist und an mindestens einer zusätzlichen Mulde, die eine Probe für den in Schritt b) verwendeten Marker enthält, adsorbiert sind.

9. Das Verfahren gemäß irgendeinem der vorhergehenden Ansprüche 1 bis 8, **dadurch gekennzeichnet, dass** es einen vorausgehenden Schritt zum Extrahieren der gesamten RNA von den Zellen, die im Harn vorhanden sind, umfasst.

10. Eine Vorrichtung zur Diagnose oder zur Überwachung von Blasentumor, **dadurch gekennzeichnet, dass** sie folgendes umfasst:
- einen Botenstoff RNA-Marker für die katalytische Komponente von Telomerase (hTRT), einen β-Actin Marker, um RNA-Zugänglichkeit nachzuweisen und als Maßstab für quantitative Schätzung, und einen zusätzlichen Marker, der ein Lymphozytenmarker ist, der geeignet ist, Entzündungszellen festzustellen, die mit neoplastischer Infiltration assoziiert sind, und optional ein Proteinmarker aus der Cytokeratin-Familie; wobei der Lymphozytenmarker, der geeignet ist, Entzündungszellen festzustellen, die mit neoplastischer Infiltration assoziiert sind, CD4 ist.

11. Die Vorrichtung gemäß Anspruch 10, **dadurch gekennzeichnet, dass** sie weiterhin eine Mehrzahl von ELISA-Platten umfasst.

12. Die Vorrichtung gemäß Anspruch 11, **dadurch gekennzeichnet, dass** sie 4 ELISA-Platten, von denen jede eine der vier spezifischen Proben umfasst: Telomerase, CK20, CD4 und β-Actin.

13. Die Vorrichtung gemäß irgendeinem der Ansprüche 10 bis 12, **dadurch gekennzeichnet, dass** sie weiterhin Streptavidin umfasst, mit dem alkalische Phosphatase oder Peroxydase in Verbindung stehen.

14. Die Vorrichtung gemäß irgendeinem der Ansprüche 10 bis 13, **dadurch gekennzeichnet, dass** sie weiterhin eine oder mehrere Waschlösungen umfasst.

15. Die Vorrichtung gemäß irgendeinem der Ansprüche 10 bis 14, **dadurch gekennzeichnet, dass** sie weiterhin eine Lösung für Hybridisierung und ein kolorimetrisches Substrat umfasst.

16. Die Vorrichtung gemäß irgendeinem der Ansprüche 10 bis 12 und 14-15, **dadurch gekennzeichnet, dass** sie weiterhin Streptavidin umfasst, dass mit Fluoreszin zur Fluoreszenzfeststellung konjugiert ist.

## Revendications

1. Méthode pour le diagnostic précoce d'une tumeur de la vessie dans un échantillon d'urine, **caractérisée en ce qu'**elle comprend la détermination, sur le ARN extrait des cellules présentes dans l'urine, de:
- un marqueur pour le ARN messager du composant catalytique de la télomérase (hTRT) et un marqueur, de préférence β-actine, pour démontrer l'accessibilité de l'ARN et comme standard pour une estimation quantitative,
en association avec un marqueur additionnel qui est un marqueur lymphocyte approprié à détecter des cellules inflammatoires associées à une infiltration néoplastique, ledit marqueur lymphocyte approprié à détecter des cellules inflammatoires associées à l'infiltration néoplastique étant CD4, et de façon optionnelle un marqueur pour une protéine de la famille des cytokératines, ledit marqueur de protéine de la famille des cytokératines étant la cytokératine 20 ou CK20.

2. Méthode pour le diagnostic précoce d'une tumeur de la vessie dans un échantillon d'urine, comprenant:
a) une phase d'amplification de l'ARN extrait des cellules présentes dans l'urine par l'utilisation de:
- un marqueur pour le ARN messager du composant catalytique de la télomérase (hTRT), un marqueur pour la β-actine pour démontrer l'accessibilité de l'ARN et comme standard pour une estimation quantitative,
en combinaison avec un marqueur additionnel qui est un marqueur lymphocyte approprié à détecter des cellules inflammatoires associées à une infiltration néoplastique, ledit marqueur lymphocyte approprié à détecter des cellules inflammatoires associées à l'infiltration néoplastique étant CD4, et de façon optionnelle un marqueur pour une protéine de la famille des cytokératines, ledit marqueur de protéine étant la cytokératine 20 ou CK20;
b) une phase de détection du matériel amplifié.

3. Méthode selon la revendication 2, **caractérisée en ce que** ladite amplification comprend:
i) la transcription inverse par utilisation d'un amorceur antisens spécifique;
ii) l'amplification par utilisation d'amorceurs sens spécifiques.

4. Méthode selon la revendication 2, **caractérisée en ce que** lesdits amorceurs sense spécifiques de la phase d'amplification ii) sont de préférence biotinylatés afin de faciliter la phase de détection consécutive.

5. Méthode selon la revendication 2, **caractérisée en ce que** ladite phase de détection comprend l'hybridation des produits amplifiés avec des sondes spécifiques aux marqueurs utilisés dans la phase b) et la reconnaissance consécutive que l'hybridation a produit avec ladite sonde de préférence au moyen de streptavidine.

6. Méthode selon la revendication 5, **caractérisée en ce que** ladite streptavidine est conjuguée à une phosphatase alcaline ou à une peroxydase pour une détection par colorimétrie ou par chimiluminescence.

7. Méthode selon la revendication 5, **caractérisée en ce que** ladite streptavidine est conjuguée à une fluorescéine pour une détection par fluorescence.

8. Méthode selon la revendication 2, **caractérisée en ce que** lesdits produits d'amplification de la phase b) sont absorbés sur un premier puits qui contient une sonde qui est immobilisée pour la hTRT et sur au moins un puits additionnel qui contient une sonde pour le marqueur utilisé dans la phase b),

9. Méthode selon l'une quelconque des revendications précédentes 1 à 8, **caractérisée en ce qu'**elle comprend une phase préliminaire d'extraction de tout l'ARN des cellules qui sont présentes dans l'urine.

10. Appareil pour le diagnostic ou pour le monitorage d'une tumeur de la vessie, **caractérisé en ce qu'**il comprend:
-- un marqueur pour l'ARN messager du composant catalytique de la télomérase (hTRT), un marqueur β-actine, pour démontrer l'accessibilité de l'ARN et comme standard pour une estimation quantitative, et un marqueur additionnel qui est un marqueur lymphocyte approprié à détecter des cellules inflammatoires associées à une infiltration néoplastique, et de façon optionnelle un marqueur de protéine de la famille des cytokératines; ledit marqueur lymphocyte approprié à détecter des cellules inflammatoires associées à une infiltration néoplastique étant CD4.

11. Appareil selon la revendication 10, **caractérisé en ce qu'**il comprend en outre une pluralité de plaques ELISA.

12. Appareil selon la revendication 11, **caractérisé en ce qu'**il comprend 4 plaques ELISA, chacune desquelles comprend l'une des quatre sondes spécifiques: télomérase, CK20, CD4 et β-actine.

13. Appareil selon l'une quelconque des revendications 10 à 12, **caractérisé en qu'**il comprend en outre de la streptavidine à laquelle est liée de la phosphatase alcaline ou de la peroxydase.

14. Appareil selon l'une quelconque des revendications 10 à 13, **caractérisé en ce qu'**il comprend en outre une ou plusieurs solutions de lavage.

15. Appareil selon l'une quelconque des revendications 10 à 14, **caractérisé en ce qu'**il comprend en outre une solution pour l'hybridation et un substrat colorimétrique.

16. Appareil selon l'une quelconque des revendications 10 à 12, 14 et 15, **caractérisé en ce qu'**il comprend en outre de la streptavidine conjuguée à de la fluorescéine pour une détection par fluorescence, ,
